Europäisches Patentamt

European Patent Office

Office européen des brevets

(19)

(11) Veröffentlichungsnummer: **0 341 559**
**A2**

(12) ## EUROPÄISCHE PATENTANMELDUNG

(21) Anmeldenummer: 89107970.9

(22) Anmeldetag: 03.05.89

(51) Int. Cl.⁴: **A61K 45/06 , A61K 31/55 ,**
**//(A61K31/55,31:135,31:44,**
**31:47,31:415,31:535,31:52,**
**31:27,31:17)**

(30) Priorität: 06.05.88 DE 3815480

(43) Veröffentlichungstag der Anmeldung:
15.11.89 Patentblatt 89/46

(84) Benannte Vertragsstaaten:
AT BE CH DE ES FR GB GR IT LI LU NL SE

(71) Anmelder: BOEHRINGER INGELHEIM KG

D-6507 Ingelheim am Rhein(DE)

(84) BE CH DE ES FR GR IT LI LU NL SE AT

Anmelder: BOEHRINGER INGELHEIM
INTERNATIONAL G.M.B.H.

D-6507 Ingelheim am Rhein(DE)

(84) GB

(72) Erfinder: Heuer, Hubert, Dr.
Am Alten Weg 29
D-6500 Mainz 32(DE)

(54) **Synergistische Kombinationen und ihre Verwendung als Therapeutika.**

(57) Bei der Anwendung von ß₂-Mimetika gemeinsam mit PAF-Antagonisten bei der Behandlung des Asthma bronchiale wird ein synergistischer Effekt erzielt.

EP 0 341 559 A2

## Synergistische Kombinationen und ihre Verwendung als Therapeutika

Die Erfindung betrifft neue Wirkstoffkombinationen aus $\beta_2$-Mimetika und PAF-Antagonisten. Durch die gemeinsame Anwendung der Wirkstoffe lassen sich vorteilhafte synergistische Wirkungen bei der Behandlung des Asthma bronchiale erreichen.

Es ist bekannt, daß $\beta_2$-Mimetika zur Behandlung des Asthma bronchiale geeignet sind, insbesondere auch zur Behandlung einer akuten asthmatischen Reaktion. Den durch PAF-induzierten Bronchospasmus sowie die durch PAF bedingten Veränderungen an der Lunge, wie mikrovasculären Leakage, Spätphasenreaktion, Zellinfiltration (Eosinophilie), Störungen der mucociliären Clearance, beeinflussen $\beta$-Mimetika jedoch kaum oder gar nicht.

Der plättchenaktivierende Faktor (PAF) hat nach dem heutigen Kenntnisstand als Mediator Bedeutung im Rahmen der Pathophysiologie des Asthma bronchiale; er soll viele Symptome des Asthma bronchiale induzieren und nachahmen können, insbesondere bronchiale Hyperreaktivität, Entzündung der Atemwege mit Oedembildung und Infiltration von Entzündungszellen (Eosinophilie), Störung der Mucussekretion und der mucociliären Clearance und Spätphasenreaktion. An der Induktion eines akuten Bronchospasmus ist PAF nach dem heutigen Stand der Erkenntnis nicht direkt beteiligt.

Überraschenderweise wurde nun gefunden, daß $\beta_2$-Mimetika und PAF-Antagonisten bei der Asthmatherapie synergistisch wirken.

Als $\beta_2$-Mimetika können die literaturbekannten Verbindungen dieser Wirkungsrichtung verwendet werden.

Hervorzuheben sind

(a) 1-(3,5-Dihydroxylphenyl)-1-hydroxy-2-[(4-hydroxyphenyl)isopropylamino]ethan (Fenoterol)

(b) 4-Amino-$\alpha$-[tert.-butylamino)methyl]-3,5-dichchlorbenzylalkohol (Clenbuterol)

(c) 2-hydroxymethyl-3-hydroxy-6-(1-hydroxy-2-tert-butylaminoethyl)pyridin (Pirbuterol)

(d) 8-Hydroxy-5-[1-hydroxy-2-[(1-methylethyl) = amino]butyl]-2(1H)-chinolinon(Procaterol)

(e) 2-(tert.-Butylamino)-1-(4-hydroxy-3-hydroxy = methylphenyl)ethanol (Salbutamol)

(f) 1-(3,5-Dihydroxyphenyl)-2-(tert.-butylamino) ethanol (Terbutalin)

(g) 1-(2-Fluor-4-hydroxylphenyl)-2-[4-(1-benzimidazolyl)-2-methyl-2-butylamino]ethanol

(h) erythro-5'-Hydroxy-8'-(1-hydroxy-2-isopropylaminobutyl)-2H-1,4-benzoxazin-3(4H)-on

(i) 1-(4-Amino-3-chlor-5-trifluormethylphenyl)-2-(tert.-butylamino)ethanol

(k) 1-(4-Ethoxycarbonylamino-3-cyan-5-fluorphenyl)-2-(tert.-butylamino)ethanol

(l) N,N'-Bis[2-(3,4-dihydroxyphenyl)-2-hydroxyethyl]hexamethylendiamin (Hexoprenalin)

(m) 7-[3-[[2-(3,5-Dihydroxyphenyl)-2-hydroxyethyl] amino]propyl]-3,7-dihydro-1,3-dimethyl-1H-purin-2,6-dion (Reproterol)

(n) [5-[2-[(1,1-Dimethylethyl)amino]-1-hydroxyethyl]-2-hydroxyphenyl]-harnstoff (Carbuterol)

(o) 2-Chlor-$\alpha$-[[(1,1-dimethylethyl)-amino]-methyl] benzylalkohol (Tulobuterol).

(p) 3-Formylamino-4-hydroxy-$\alpha$[N-[1-methyyl-2-p-methoxyphenyl)ethyl]-aminomethyl]benzylalkohol semifumarat (Formoterol)

(g) N-[2-Hydroxy-5-[1-hydroxy-2-[[2-(4-methoxyphenyl)-1-methylethyl]amino]ethyl]phenyl]-formamid semifumarat

(r) 2-Hydroxymethyl-3-hydroxy-6-[1-hydroxy-2-tert. butylaminoethyl)-pyridin dihydrochlorid (Pirbuterol)

(s) $\alpha$[(tert.Butylamino)-methyl-4-hydroxy-3(methylsulfonyl)methyl]benzylalkohol hydrochlorid (Sulfonterol)

(t) 5-[2-(tert.-Butylamino)-1-hydroxyethyl]-m-phenylen-bis dimethylcarbamat) (Bambuterol)

(u) 5-[2-(tert.-Butylamino)-1-hydroxyethyl]-m-phenylendiisobutyrat (Ibuterol) (v) 4-[2-(tert.-Butylamino)-1-hydroxyethyl]-1,2-phenylen-di-4-toluat (Bitolterol)

(w) 4-Hydroxy-$\alpha$[[(6-(4-phenylbutoxy]hexylamino]methyl-m-xylen-$\alpha,\alpha'$ diol (Salmetrol)

PAF-Antagonisten sind in großer Zahl beschrieben worden, beispielsweise in den Europa-Anmeldungen 176 927, 176 928, 176 929, 194 416, 230 942, 240 899, 254 245, 255 028, auf deren Inhalt Bezug genommen wird.

Die in diesen Veröffentlichungen offenbarten PAF-Antagonisten eignen sich für die Anwendung gemäß der Erfindung. Hervorzuheben ist die Verwendung von 4-(2-Chlorphenyl)-9-methyl-2-[3-(4-morpholinyl)-3-propanon-1-yl]-6H-thieno[3,2-f][1,2,4]-triazolo[4,3-a] [1,4]diazepin (WEB 2086) oder 6-(2-Chlorphenyl)-8,9-dihydro-1-methyl-8-[(4-morpholinyl)carbonyl]- 4H, 7H-cyclopenta[4,5]thieno[3,2-f][1,2,4]triazolo(4,3-a][1,4]-diazepin (WEB 2170) (6-(2-Chlorphenyl)-8,9-dihydro-1-methyl-8-[dipropylaminocarbonyl]-4H,7H-cyclopenta-

[4,5]thieno[3,2-f][1,2,4]triazolo[4,3-a][1,4]diazepin (Web 2347) oder ihrer Säureadditionssalze sowie der in den oben genannten Europaanmeldungen hervorgehobenen Verbindungen.

Die überraschenden Vorteile der neuen Kombinationen bestehen vor allem darin, daß gegenüber der alleinigen Anwendung von ß2-Mimetika eine geringere Dosis dieser Wirkstoffe ausreicht, wodurch störende Nebenwirkungen der ß2-Mimetika reduziert werden, außerdem ist ein leichteres Ansprechen auf die Therapie festzustellen.

Die Dosis an ß2-Mimetikum und PAF-Antagonist liegt jeweils zwischen 20 % und 95 % derjenigen Dosis, die bei alleiniger Anwendung der Wirkstoffe benutzt wird, wobei vorzugsweise eine höhere Dosierung der einen Komponente (im Rahmen der obigen Angaben) mit einer geringeren Dosierung der anderen Komponente verknüpft wird.

Die erfindungsgemäßen Kombinationen können in gemeinsamen Formulierungen oder in Form von getrennten Formulierungen in kurzem Zeitabstand verabreicht werden.

Die Formulierung zu gebräuchlichen Anwendungsformen, etwa Tabletten, Dragées, Granulaten, Kapseln, Injektionslösungen, Dosieraerosolen, Inhalationspulvern, erfolgt in an sich bekannter Weise und mit bekannten HIIFS- und/oder Trägerstoffen.

## 1. Tabletten

| 5 Gew.-Teile | Wirkstoffkombination gemäß der Erfindung |
|---|---|
| 1 Gew.-Teile | Stearinsäure |
| 194 Gew.-Teile | Traubenzucker |

Die Bestandteile werden zu Tabletten von 200 mg verpreßt.

## 2. Inhalationsaerosol

| 0,02 Gew.-Teile | Fenoterolhydrobromid |
|---|---|
| 0,10 Gew.-Teile | WEB 2086 |
| 0,20 Gew.-Teile | Sojalecethin |
| ad 100,00 Gew.-Teile | Treibgas (Frigen 11, 12 und 114). |

Die Mischung wird in Aerosolbehälter mit Dosierventil gefüllt, der einzelene Hub wird so bemessen, daß eine Dosis von 0,12 mg der Wirkstoffkombination abgegeben wird.

## Pharmakologische Untersuchungen

Propranolol (0,1 mg/kg i.v.) steigert die Empfindlichkeit von NMRI-Mäusen gegenüber PAF (30 ng/kg i.v.); die Wahrscheinlichkeit eines PAF-induzierten tödlichen Schocks steigt von 0 % auf 93 %. Diese Wirkung kann dosisabhängig durch ß2-Mimetika oder PAF-Antagonisten verhindert bzw. vermindert werden.

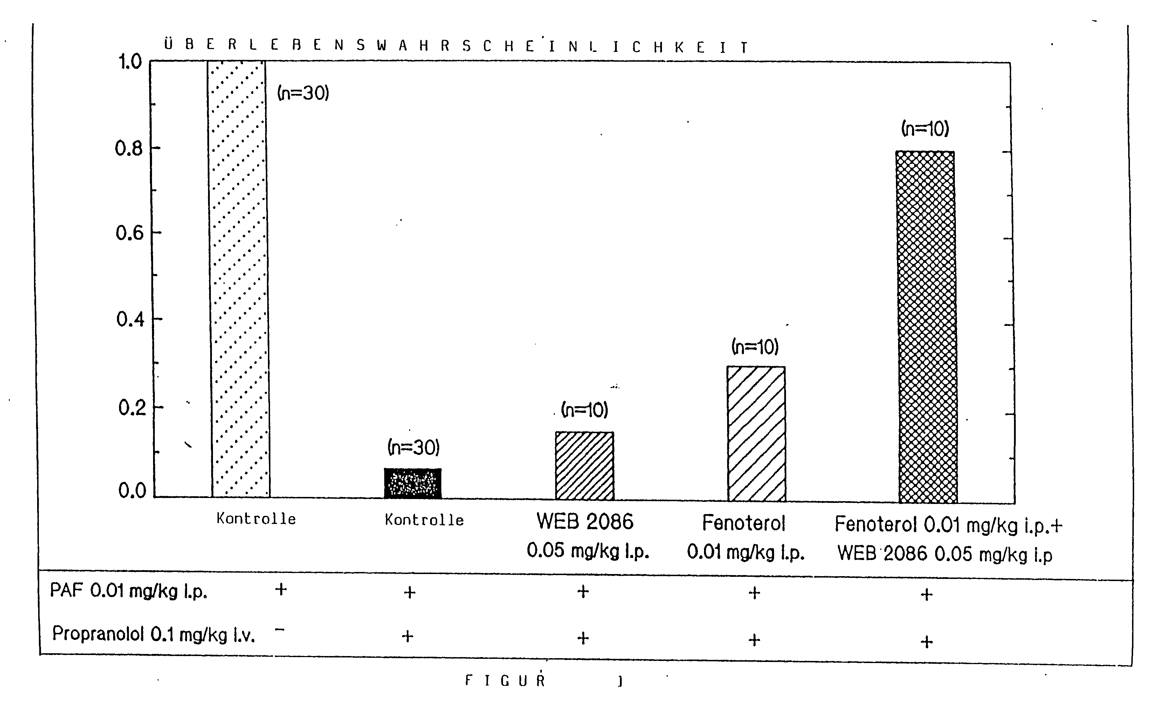

Wie Figur 1 zeigt, wird z.B. die Überlebenswahrscheinlichkeit bei der gleichzeitigen Anwendung von Fenoterol und WEB 2086 fast auf das Doppelte gegenüber der Summe der Einzelwirkungen gesteigert.

## Ansprüche

1. Synergistisches Mittel zur Behandlung des Asthma bronchiale, dadurch gekennzeichnet, daß es als Wirkstoffe mindestens ein ß2-Mimetikum und mindestens einen PAF-Antagonisten enthält.

2. Mittel nach Anspruch 1, dadurch gekennzeichnet, daß das ß2-Mimetikum Fenoterol, Clenbuterol, Pirbuterol, Procaterol, Salbutamol, Tertutalin, Hexooprenalin, Reproterol, Carbuterol, Tulobuterol, 1-(2-Fluor-4-hydroxylphenyl)-2-[4-(1-benzimidazolyl)-2-methyl-2-butylamino]-ethanol, erythro-5´-Hydroxy-8´-(1-hydroxy-2-isopropylaminobutyl)-2H-1,4-benzoxazin-3(4H)-on, 1-(4-Amino-3-chlor-5-trifluormethylphenyl)-2-(tert.-butylamino)ethanol oder 1-(4-Ethoxycarbonylamino- 3-cyan-5-fluorphenyl)-2-(tert.-butylamino)ethanol Formoterol, Sulfonterol, Bambuterol, Ibuterol, Bitolterol, Salmetrol ist.

3. Mittel nach Anspruch 1 oder 2, dadurch gekennzeichnet, daß der PAF-Antagonist WEB 2086, Web 2347 oder WEB 2170, gegebenenfalls in Form eines Säureadditionssalzes, ist.

4. Verwendung von ß2-Mimetika in Kombination mit PAF-Antagonisten zur Herstellung eines Arzneimittels zur Behandlung des Asthma bronchiale.

5. Verwendung von Fenoterol, Clenbuterol, Pirbuterol, Procaterol, Salbutamol, Tertutalin, Hexooprenalin, Reproterol, Carbuterol, Tulobuterol, 1-(2-Fluor-4-hydroxylphenyl)-2-[4-(1-benzimidazolyl)-2-methyl-2-butylamino]-ethanol, erythro-5´-Hydroxy-8´-(1-hydroxy-2-isopropylaminobutyl)-2H-1,4-benzoxazin-3(4H)-on, 1-(4-Amino-3-chlor- 5-trifluormethylphenyl)-2-(tert.-butylamino)ethanol oder 1-(4-Ethoxycarbonylamino-3-cyan-5-fluorphenyl)-2-(tert.-butylamino)ethanol Formoterol, Pirbuterol, Sulfonterol, Bambuterol, Ibuterol, Bitolterol, Salmetrol in Kombination mit PAF-Antagonisten zur Herstellung eines Arzneimittels zur Behandlung des Asthma bronchiale.

6. Verwendung nach Anspruch 4 oder 5, dadurch gekennzeichnet, daß der PAF-Antagonist WEB 2086, Web 2347 oder WEB 2170 ist, gegebenenfalls in Form eines Säureadditionssalzes, ist.

ÜBERLEBENSWAHRSCHEINLICHKEIT

| | Kontrolle | Kontrolle | WEB 2086 0.05 mg/kg i.p. | Fenoterol 0.01 mg/kg i.p. | Fenoterol 0.01 mg/kg i.p.+ WEB 2086 0.05 mg/kg i.p |
|---|---|---|---|---|---|
| PAF 0.01 mg/kg i.p. | + | + | + | + | + |
| Propranolol 0.1 mg/kg i.v. | − | + | + | + | + |

FIGUR 1

EP 0 341 559 A2